# EUROPEAN PATENT SPECIFICATION

(11) **EP 2 580 206 B1**
(45) Date of publication and mention of the grant of the patent: **23.09.2015**
(21) Application number: 11792704.6
(22) Date of filing: 10.06.2011
(51) Int. Cl.: C07D 401/12, C07D 401/14, A61K 31/497, A61P 35/00

(54) **PHARMACEUTICAL COMPOSITION COMPRISING AMIDE DERIVATIVE OR PHARMACEUTICALLY ACCEPTABLE SALT THEREOF**
PHARMAZEUTISCHE ZUSAMMENSETZUNG MIT EINEM AMIDDERIVAT ODER EINEM PHARMAZEUTISCH ZULÄSSIGEN SALZ DAVON
COMPOSITION PHARMACEUTIQUE COMPRENANT UN DÉRIVÉ D'AMIDE OU SON SEL PHARMACEUTIQUEMENT ACCEPTABLE

(30) Priority: 11.06.2010 KR 20100055549
(43) Date of publication of application: 17.04.2013
(73) Proprietor: Hanmi Science Co., Ltd., Gyeonggi-do 445-813 (KR)
(72) Inventor: KIM, Yong Il, Suwon-si Gyeonggi-do 442-718 (KR); KIM, Kyeong Soo, Suwon-si Gyeonggi-do 442-150 (KR); JANG, Ki Young, Suwon-si Gyeonggi-do 440-330 (KR); KIM, Yo Han, Seoul 151-058 (KR); PARK, Jae Hyun, Suwon-si Gyeonggi-do 443-470 (KR); WOO, Jong Soo, Suwon-si Gyeonggi-do 440-710 (KR)
(74) Representative: Towler, Philip Dean
(86) International application number: PCT/KR2011/004271
(87) International publication number: WO 2011/155793

(56) References cited:
- WO-A1-2005/030757
- WO-A2-2008/150118
- KR-A- 980 008 219
- KR-A- 20000 048 572
- KR-A- 20020 032 612
- KR-A- 20080 107 294
- US-A1- 2007 037 837

## Description

### FIELD OF THE INVENTION

The present invention relates to a pharmaceutical composition comprising an amide derivative or a pharmaceutically acceptable salt thereof with improved storage stability.

### BACKGROUND OF THE INVENTION

There are many signal transduction systems in cells which are functionally linked to each other to control the proliferation, growth, and apoptosis of cells (Kaelin, Nature Reviews Cancer, 2005, 5:689). The breakdown of the intracellular controlling system by genetic or environmental factors causes abnormal amplification or destruction of the signal transduction leading to tumor cell generation (Hanahan and Weinberg, Cell, 2000, 100:57).

Protein tyrosine kinases play important roles in such intracellular signal transduction (Melnikova and Golden, Nature Revieus Drug Discovery, 2004, 3:993), and their abnormal expression or mutation has frequently been observed in cancer cells. A protein tyrosine kinase is an enzyme which catalyzes the transportation of a phosphate group from ATP to tyrosine on protein substrates. Many growth factor receptor proteins function as a tyrosine kinase to transport cellular signals. The interaction between growth factors and their receptors is essential to normal control of the cellular growth, but abnormal signal transduction caused by the mutation or overexpression of such receptors often induces tumor cells and cancers.

Protein tyrosine kinases have been classified into many families in accordance with their growth factor types, and epithelial cell growth factor (EGF) and its EGF receptor (EGFR) tyrosine kinase, in particular, have been intensely studied (Hynes and Lane, Nature Reviews Cancer, 2005, 5:341). An EGFR tyrosine kinase, a transmembrane protein, is composed of a receptor and a tyrosine kinase, and delivers extracellular signals to the cell nucleus through the cellular membrane. Various EGFR tyrosine kinases are classified, based on their structural characteristics, into EGFR (Erb-B1), Erb-B2, Erb-B3 and Erb-B4, each of which can form a homodimer- or heterodimer-signal delivery complex together with other subtypes. It has been reported that overexpression of two or more of such heterodimers in malignant diseases may result in increased transmutation. Such overexpression is often observed in malignant tumors.

Gefitinib or Erlotinib, developed as small molecules for the inhibition of EGFR tyrosine kinases, selectively and reversibly inhibits EGFR (Erb-B1), a subtype of EGFR, and has been used as a treating agent for non-small cell lung carcinoma (NSCLC). Lapatinib, approved as a treating agent for breast cancer by the U.S. Food and Drug Administration (FDA) in 2007, reversibly inhibits both Erb-B1 and Erb-B2 among the EGFR subtypes.

Also, several drugs for inhibiting EGFR tyrosine kinases, e.g., irreversible inhibitors such as Canertinib, HKI-272, BIBW-2992 and PF00299804, and reversible inhibitors such as AEE-788, CP24714, ARRY334543 and AV-412, are currently under clinical trials. These inhibitors are developed to selectively inhibit Erb-B2, simultaneously inhibit two or more EGFR subtypes including Erb-B1, or EGFR and other receptors.

From the clinical test results for treating NSCLC, these irreversible inhibitors showed improved drug activity compared to the conventional reversible inhibitors. However, such drugs provide no significant therapeutic effects to patients suffering from drug-resistant cancer.

Therefore, there has been a continued need to develop a novel drug effective in treating such resistant cancer compared to the conventional irreversible inhibitors while causing no adverse side effects. In accordance with the need, the present inventors developed a novel compound which selectively and effectively inhibits the growth of cancer cells and the development of drug resistance induced by the EGFR and EGFR mutants without side effects, as disclosed in Korea Patent Laid-open Publication No. 2008-0107294.

The present inventors have also developed various forms of formulation comprising 1-(4-(4-(3,4-dichloro-2-fluorophenylamino)-7-methoxyquinazolin-6-yloxy)piperidin-1-yl)prop-2-en-1-on of formula (I) disclosed in Korea Patent Laid-open Publication No. 2008-0107294 as an active ingredient. However, the formulation prepared by using the conventional pharmaceutical acceptable additives caused a lowered amount of the compound and facilitated the formation of a compound of formula (II) (Impurity E) during a storage period.

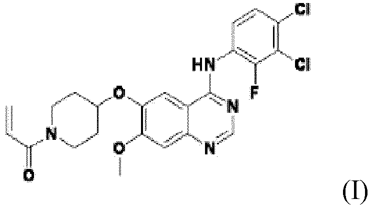

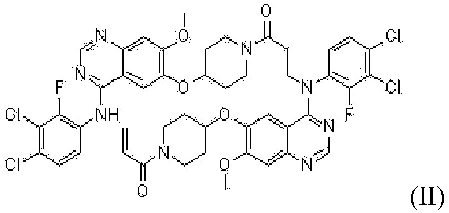

A purity of the active ingredient is an important factor for preparing a safe and effective pharmaceutical composition. Especially, anticancer drugs applied to the patients suffering from cancer having very weak immune system are required to comprise an active ingredient with a maximum purity. Impurities of a drug such as degraded products from the final drug product induced by various environmental factors such as temperature, humidity and light in addition to impurities capable of being removed in the preparation process of the active ingredient may cause various side effects to the patients who are being treated.

Hence, the present inventors have endeavored to improve the stabilities of the compound of formula (I) by inhibiting the formation of any impurities, and found that an acidic additive is effective in improving the stabilities of the inventive active ingredient.

### SUMMARY OF THE INVENTION

It is an object of the present invention to provide a pharmaceutical composition comprising an amide derivative or a pharmaceutically acceptable salt thereof with improved stability.

In accordance with one aspect of the present invention, there is provided a pharmaceutical composition comprising a compound of formula (I) or a pharmaceutically acceptable salt thereof, and an acidic additive: 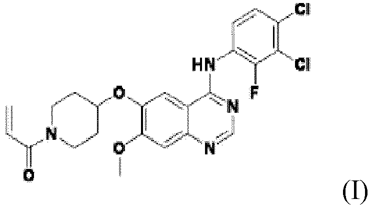 wherein said acidic additive is alginic acid or SiO₂.

### BRIEF DESCRIPTION OF THE DRAWINGS

The above and other objects and features of the present invention will become apparent from the following description of the invention, when taken in conjunction with the accompanying drawings, which respectively show:
Fig. 1: the stabilities at 60 °C of the formulations prepared in accordance with Examples 1 to 7, and Comparative Example 1;
Fig. 2: the stabilities of the formulations according to the amount of acidic additive;
Fig. 3: the stabilities at 60 °C of the formulations prepared in accordance with Reference Example 1, and Comparative Examples 2 to 5; and
Fig. 4: the stabilities at 60 °C of the formulations prepared in accordance with Reference Examples 11 and 12, and Comparative Example 6.

### DETAILED DESCRIPTION OF THE INVENTION

The present invention provides a pharmaceutical composition comprising a compound of formula (I) or a pharmaceutically acceptable salt thereof, and an acidic additive: 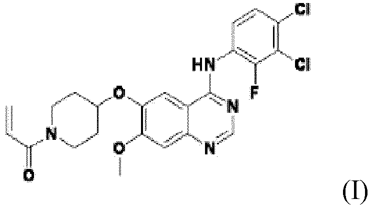 wherein said acidic additive is alginic acid or SiO₂.

The pharmaceutical composition according to the present invention improves the stability of the active ingredient by employing an acidic additive and inhibiting impurity formation. The pharmaceutical composition comprising the compound of formula (I) as the active ingredient shows low stability due to impurities formed during the storage period. However, the inventive pharmaceutical composition prepared by employing an acidic additive that is alginic acid or SiO₂ can more effectively inhibit the impurity formation compared to a pharmaceutical composition prepared by reducing the water content, or adding a particular excipient or stabilizer in the preparation process.

Each ingredient of the inventive pharmaceutical composition is described in detail as follows.

### (1) Active ingredient

The active ingredient used in the pharmaceutical composition of the present invention is 1-(4-(4-(3,4-dichloro-2-fluorophenylamino)-7-methoxyquinazolin-6-yloxy)piperidin-1-yl)prop-2-en-1-on of formula (I) or a pharmaceutically acceptable salt thereof.

The compound of formula (I) has been disclosed as a novel compound which can selectively and effectively inhibit the growth of cancer cells and the development of drug resistance induced by EGFR and EGFR mutants, while causing no adverse side effects (*see* Korean Patent Laid-open Publication No. 2008-0107294).

In the present invention, the pharmaceutically acceptable salts include, but not limited to, an acid-addition salt of an inorganic acid or organic acid, or a metal salt. Preferably, examples of the inorganic acid-addition salt may include salts of hydrochloric acid, phosphoric acid, sulfuric acid, or disulfuric acid; examples of the organic acid salt may include salts of malic acid, maleic acid, citric acid, fumaric acid, besylic acid, camsylic acid, or edisylic acid; and examples of the metal salts may include calcium salt, sodium salt, magnesium salt, strontium salt, or potassium salt.

An amount ranging from 0.1 mg to 1,000 mg of the active ingredient of the present invention may be included in the composition per 1 formulation unit.

### (2) Acidic additive

The acidic additive used in the pharmaceutical composition of the present invention is alginic acid or SiO₂.

Alginic acid and SiO₂ are materials showing a pH of 1 to 6 when dissolved or dispersed in water at a concentration of 1 to 5% (w/v).

In the pharmaceutical composition of the present invention, the acidic additive may be employed in an amount ranging from 0.1 to 100 parts by weight, preferably, 0.25 to 50 parts by weight, based on 1 part by weight of the compound of formula (I).

The acidic additive may be added during the wet or dry granulating process of the compound of formula (I) and a pharmaceutically acceptable excipient (e.g., a disintegrant, a lubricant, a diluent and the like), or even after the granulating process. Also, it may be added to a mixture of the compound of formula (I) and a pharmaceutically acceptable excipient for direct compression or filling a capsule.

### (3) Other ingredients

The pharmaceutical composition of the present invention may further comprise at least one excipient selected from a diluent, a binder, a disintegrant, and a lubricant. Examples of the diluent may include microcrystalline cellulose, lactose, mannitol, calcium phosphate and the like; examples of the binder may include povidone, hydroxypropyl cellulose, 1-hydroxypropyl methylcellulose, polyvinyl alcohol, sodium carboxymethyl cellulose and the like; examples of the disintegrant may include crospovidone, sodium croscarmellose, sodium starch glycolate and the like; and examples of the lubricant may include magnesium stearate, calcium stearate, sodium stearyl fumarate and the like.

Preferably, the diluent may be used in an amount ranging from 20 to 80% by weight, the binder may be used in an amount ranging from 1 to 10% by weight, the disintegrant may be used in an amount ranging from 1 to 30% by weight and the lubricant may be used in an amount ranging from 0.5 to 5% by weight, based on the total weight of the composition.

The pharmaceutical composition of the present invention can be formulated for oral administration. Representative examples of the formulation for oral administration may include powders, a tablet, a capsule, granules or syrup, preferably a tablet or capsule, but are not limited thereto.

The pharmaceutical composition of the present invention may be coated with a coating substrate to prevent the composition from being in contact with the hand or skin of a user. The coating substrate employed in the present invention may include a rapid release coating substrate such as 1-hydroxypropyl cellulose, 1-hydroxypropyl methylcellulose, polyvinyl alcohol, polyvinyl alcohol-polyethylene glycol graft polymer (Kollocoat® IR, BASF) and the like; an enteric coating substrate such as (metha)acrylate copolymer (Eudragit®, EVONIK), hydroxypropyl methylcellulose phthalate, cellulose acetate phthalate and the like; and a sustained release coating substrate such as cellulose acetate, ethyl cellulose, polyvinyl acetate and the like. The coating substrate may be used in an amount ranging from 1 to 50% by weight, preferably 1 to 30% by weight, based on the uncoated core.

### Example

The following Examples are intended to further illustrate the present invention without limiting its scope.

### Examples 1 to 7: Preparation of a tablet comprising acidic additives

In accordance with the composition described in Table 1, a tablet having a compound of formula (I) was prepared by using a compound of formula (I) (Hanmi Pharm. Co., Ltd., Korea), mannitol, povidone (BASF, Germany), crospovidone (BASF, Germany), magnesium stearate and one or more acidic additives such as citric acid, erythorbic acid, phosphoric acid, alginic acid, stearic acid and silicon dioxide. Acidic additives (citric acid and phosphoric acid) added to a wet granule composition were dissolved or dispersed in water, the binding solution. Acidic additives were sieved through a 30 mesh, when they were added after granulation or added to a mixture of direct compression. Each of tablets of Examples 1 to 7 was prepared into a tablet having a hardness of 6 to 12 kp by using a tablet machine.

**Table 1**

| | | | Examples | | | | | | |
|---|---|---|---|---|---|---|---|---|---|
| | | | 1* | 2* | 3* | 4* | 5 | 6 | 7 |
| Wet granule | Mixture | Compound of formula (I) | 8 | 8 | 8 | 8 | 8 | 8 | 8 |
| | | Mannitol | 98 | 98 | 98 | 98 | 98 | 98 | 98 |
| | Binding solution | Povidone | 3 | 3 | 3 | 3 | 3 | 3 | 3 |
| | | Citric acid | - | 10 | - | - | | | |
| | | Phosphoric acid | | | 10 | | | | |
| | | Distilled water | 20 | 20 | 20 | 20 | 20 | 20 | 20 |
| Mixture | | Mannitol | 79 | 79 | 79 | 79 | 79 | 79 | 79 |
| | | Crospovidone | 10 | 10 | 10 | 10 | 10 | 10 | 10 |
| | | Citric acid | 10 | - | - | - | - | - | - |
| | | Erythorbic acid | - | - | - | 10 | - | - | - |
| | | Alginic acid | - | - | - | - | 10 | 5 | 5 |
| | | Stearic acid | - | - | - | - | - | 5 | - |
| | | Silicon dioxide | - | - | - | - | - | - | 5 |
| Final Mixture | | Magnesium stearate | 2 | 2 | 2 | 2 | 2 | 2 | 2 |
| Total weight excluding distilled water | | | 210 | 210 | 210 | 210 | 210 | 210 | 210 |

| | | | | | | | | | |
|---|---|---|---|---|---|---|---|---|---|
| * Reference only, not claimed | | | | | | | | | |

### Reference Examples 8 to 10: Preparation of a tablet comprising different amounts of an acidic additive

In accordance with the composition described in Table 2, each of the tablets of Examples 8 to 10 was prepared based on the procedure of Example 1 except using citric acid, as an acidic additive, with different amounts.

**Table 2**

| | | | Examples | | |
|---|---|---|---|---|---|
| | | | 8* | 9* | 10* |
| Wet granule | Mixture | Compound of formula (I) | 8 | 8 | 8 |
| | | Mannitol | 98 | 98 | 98 |
| | Binding solution | Povidone | 3 | 3 | 3 |
| | | Distilled water | 20 | 20 | 20 |
| Mixture | | Mannitol | 79 | 79 | 79 |
| | | Crospovidone | 10 | 10 | 10 |
| | | Citric acid | 2 | 5 | 20 |
| Final mixture | | Magnesium stearate | 2 | 2 | 2 |
| Total weight excluding distilled water | | | 202 | 205 | 220 |

| | | | | | |
|---|---|---|---|---|---|
| * Reference only, not claimed | | | | | |

### Comparative Examples 1 to 5: Preparation of a tablet comprising a non-acidic antioxidant or a basic stabilizer

In accordance with the composition described in Table 3, a tablet of Comparative Examples 1 was prepared based on the procedure of Example 1 without using any acidic additive. Further, in accordance with the composition described in Table 3, each of the tablets of Comparative Examples 2 to 5 was prepared based on the procedure of Example 1 except that, instead of using an acidic additive, butylated hydroxy toluene (BHT) or tocopherol was used as a non-acidic antioxidant, or calcium carbonate (CaCO₃) or meglumine was used as a basic stabilizer.

**Table 3**

| | | | Comparative Examples | | | | |
|---|---|---|---|---|---|---|---|
| | | | 1 | 2 | 3 | 4 | 5 |
| Wet granule | Mixture | Compound of formula (I) | 8 | 8 | 8 | 8 | 8 |
| | | Mannitol | 98 | 98 | 98 | 98 | 98 |
| | Binding solution | Povidone | 3 | 3 | 3 | 3 | 3 |
| | | Tocopherol | - | 10 | - | - | - |
| | | Distilled water | 20 | 20 | 20 | 20 | 20 |
| Mixture | | Mannitol | 79 | 79 | 79 | 79 | 79 |
| | | Crospovidone | 10 | 10 | 10 | 10 | 10 |
| | | BHT | - | - | 1 | - | - |
| | | Calcium carbonate | - | - | - | 10 | - |
| | | Meglumine | - | - | - | - | 10 |
| Final mixture | | Magnesium stearate | 2 | 2 | 2 | 2 | 2 |
| Total weight excluding distilled water | | | 200 | 210 | 201 | 210 | 210 |

### Reference Examples 11 and 12, and Comparative Example 6: Preparation of a capsule

In accordance with the composition described in Table 4, the compound of formula (I) and excipients were sieved through a 30 mesh and then mixed. A mixture containing 8 mg of the compound of formula (I) was charged into a capsule of size No. 0 to prepare each of the capsules of Examples 11 and 12. A capsule of Comparative Examples 6 was prepared based on the procedure of Example 11 without adding any acidic additive.

**Table 4**

| | Example 11* | Example 12* | Comparative Example 6 |
|---|---|---|---|
| Compound of formula (I) | 8 | 8 | 8 |
| Mannitol | 97 | 97 | 97 |
| Povidone | 3 | 3 | 3 |
| Crospovidone | 10 | 10 | 10 |
| Citric acid | 10 | - | - |
| Erythorbic acid | - | 10 | - |
| Magnesium stearate | 2 | 2 | 2 |
| Total weight of the mixture | 130 | 130 | 120 |

| | | | |
|---|---|---|---|
| * Reference only, not claimed | | | |

### Experimental Example 1: Evaluation for storage stability of each of the formulations of Examples and Comparative Examples

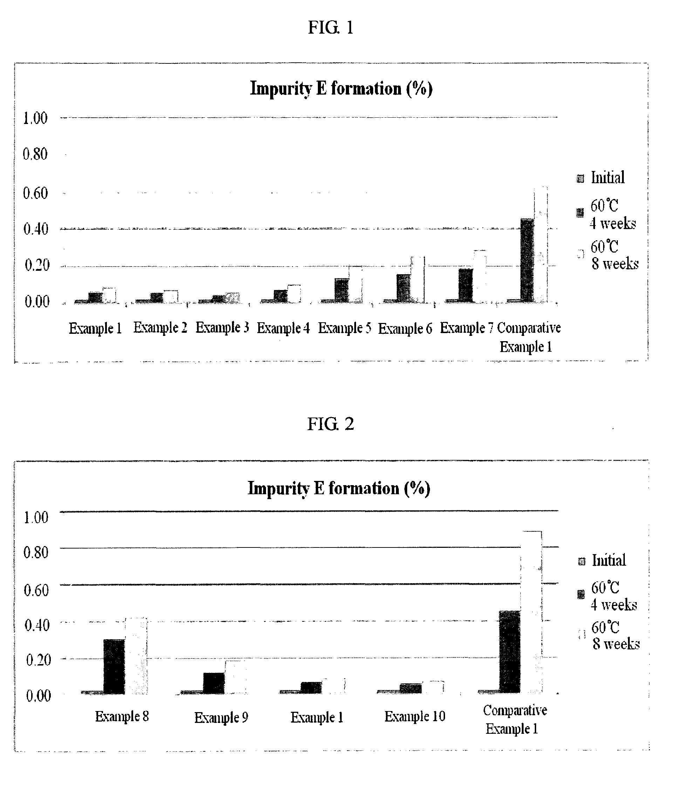

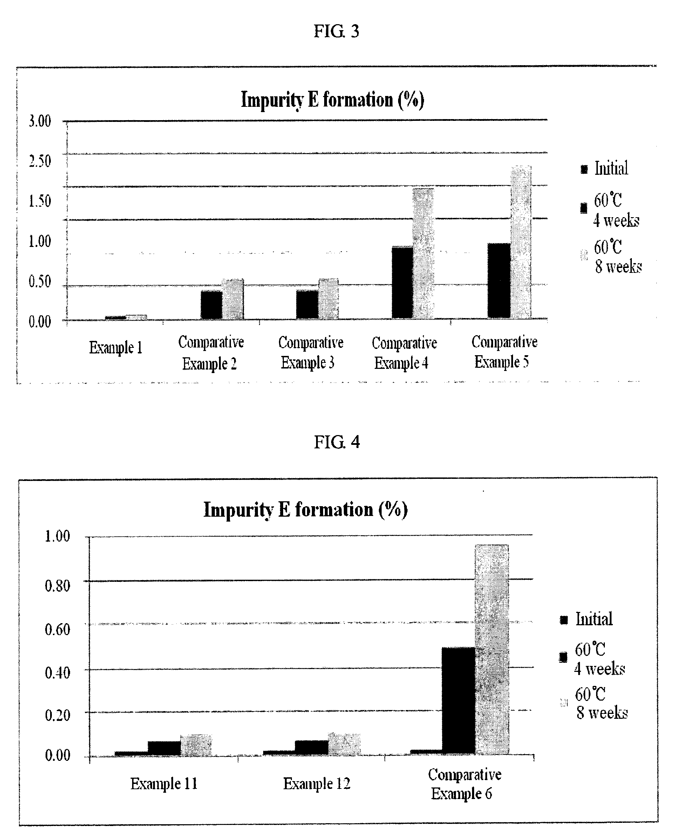

In order to evaluate storage stability of formulations containing the compound of formula (I) prepared in accordance with Examples 1 to 12 and Comparative Examples 1 to 6, the amount of the compound of formula (II) (Impurity E), as a major degradation product, was measured. The formulations were each packaged with 1 g of silica gel in an HDPE bottle, and stored in a chamber (60°C) and the amount of Impurity E was measured four and eight weeks later. The results are shown in Tables 5 to 7 and Figs. 1 to 4.

**Table 5**

| | Examples | | | | | | | | | |
|---|---|---|---|---|---|---|---|---|---|---|
| | 1* | 2* | 3* | 4* | 5 | 6 | 7 | 8* | 9* | 10* |
| Initial (0 week) | 0.02 | 0.02 | 0.02 | 0.02 | 0.02 | 0.02 | 0.02 | 0.02 | 0.02 | 0.02 |
| 4 weeks (60°C) | 0.06 | 0.05 | 0.04 | 0.07 | 0.13 | 0.15 | 0.18 | 0.30 | 0.19 | 0.05 |
| 8 weeks (60 °C) | 0.08 | 0.07 | 0.06 | 0.10 | 0.20 | 0.26 | 0.29 | 0.40 | 0.29 | 0.07 |

| | | | | | | | | | | |
|---|---|---|---|---|---|---|---|---|---|---|
| Reference only, not claimed | | | | | | | | | | |

**Table 6**

| | Comparative Example | | | | |
|---|---|---|---|---|---|
| | 1 | 2 | 3 | 4 | 5 |
| Initial (0 week) | 0.02 | 0.02 | 0.02 | 0.02 | 0.02 |
| 4 weeks (60°C) | 0.45 | 0.45 | 0.44 | 1.09 | 1.14 |
| 8 weeks (60°C) | 0.88 | 0.63 | 0.61 | 1.98 | 2.32 |

**Table 7**

| | Example 11 * | Example 12* | Comparative Example 6 |
|---|---|---|---|
| Initial (0 week) | 0.02 | 0.02 | 0.02 |
| 4 weeks (60°C) | 0.07 | 0.07 | 0.49 |
| 8 weeks (60°C) | 0.10 | 0.11 | 0.96 |

| | | | |
|---|---|---|---|
| * Reference only, not claimed | | | |

As shown in Tables 5 to 7 and Figs. 1 to 4, the production of Impurity E was reduced about 4 to 10 times or more, and thus the storage stability of the formulations containing the compound of formula (I) was improved by adding one or more acidic additives to the formulations. According to the guidelines of the International Conference on Hanmonisation of Technical Requirements for Registration of Pharmaceuticals for Human Use (ICH), the limits of unknown impurities and known impurities are respectively prescribed as 0.2% and 0.5%. In this regard, the addition of one or more acidic additives can improve the stability of the tablets and capsules containing the compound of formula (I), and thus excellent storage stability can be expected.

However, a non-acidic antioxidant (BHT or tocopherol) or a basic stabilizer (calcium carbonate (CaCO₃) or meglumine), commonly used as a pharmaceutical stabilizer, did not improve the stability or had undesirable effects on the stability.

While the invention has been described with respect to the above specific embodiments, it should be recognized that various modifications and changes may be made to the invention by those skilled in the art which also fall within the scope of the invention as defined by the appended claims.

## Claims

1. A pharmaceutical composition comprising a compound of formula (I) or a pharmaceutically acceptable salt thereof; and an acidic additive: 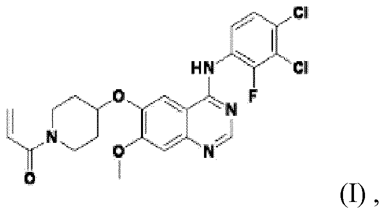 wherein said acidic additive is alginic acid or SiO₂.

2. The pharmaceutical composition of claim 1, wherein the pharmaceutically acceptable salt is an inorganic acid salt, an organic acid salt, or a metal salt.

3. The pharmaceutical composition of claim 2, wherein the inorganic acid salt is a salt of hydrochloric acid, phosphoric acid, sulfuric acid, or disulfuric acid; the organic acid salt is a salt of malic acid, maleic acid, citric acid, fumaric acid, besylic acid, camsylic acid, or edisylic acid; and the metal salt is a calcium salt, sodium salt, magnesium salt, strontium salt, or potassium salt.

4. The pharmaceutical composition of claim 1, wherein the compound of formula (I) is contained in an amount ranging from 0.1 mg to 1,000 mg per 1 unit of the composition.

5. The pharmaceutical composition of claim 1, wherein the acidic additive is contained in an amount ranging from 0.1 to 100 parts by weight based on 1 part by weight of the compound of formula (I).

6. The pharmaceutical composition of claim 5, wherein the acidic additive is contained in an amount ranging from 0.25 to 50 parts by weight based on 1 part by weight of the compound of formula (I).

7. The pharmaceutical composition of claim 1, wherein the composition is formulated in the form of a capsule or tablet.

8. The pharmaceutical composition of claim 1, which further comprises an excipient selected from the group consisting of a diluent, a binder, a disintegrant, a lubricant and a mixture thereof.

9. The pharmaceutical composition of claim 8, wherein the diluent is contained in an amount ranging from 20 to 80% by weight, the binder is contained in an amount of 1 to 10% by weight, the disintegrant is contained in an amount ranging from 1 to 30% by weight, or the lubricant is contained in an amount ranging from 0.5 to 5% by weight, based on the total weight of the composition.

10. The pharmaceutical composition of claim 1, which is coated with a rapid release coating substrate, an enteric coating substrate, a sustained release coating substrate, or a mixture thereof.

11. The pharmaceutical composition of claim 10, wherein the rapid release coating substrate is hydroxypropyl cellulose, hydroxypropyl methylcellulose, polyvinyl alcohol, polyvinyl alcohol-polyethylene glycol graft polymer or a mixture thereof; the enteric coating substrate is (metha)acrylate copolymer, 1-hydroxypropyl methylcellulose phthalate, cellulose acetate phthalate or a mixture thereof; and the sustained release coating substrate is cellulose acetate, ethyl cellulose, polyvinyl acetate or a mixture thereof.

12. The pharmaceutical composition of claim 10, wherein the coating substrate is used in an amount ranging from 1 to 50% by weight, based on the total weight of the uncoated core.

## Patentansprüche

1. Pharmazeutische Zusammensetzung, umfassend eine Zusammensetzung mit der Formel (I) oder ein pharmazeutisch unbedenkliches Salz davon; und einen sauren Zusatzstoff; 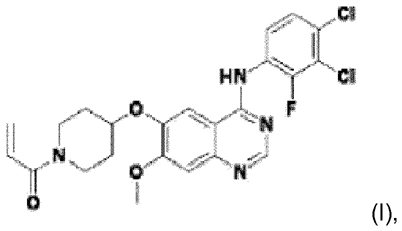 wobei der saure Zusatzstoff Alginsäure oder SiO₂ ist.

2. Pharmazeutische Zusammensetzung nach Anspruch 1, wobei das pharmazeutisch unbedenkliche Salz ein Salz einer anorganischen Säure ist, ein Salz einer organischen Säure oder ein Metallsalz ist.

3. Pharmazeutische Zusammensetzung nach Anspruch 2, wobei das Salz der anorganischen Säure ein Salz der Salzsäure, der Phosphorsäure, der Schwefelsäure oder der Dischwefelsäure ist; das organische Salz ein Salz der Äpfelsäure, der Maleinsäure, der Citronensäure, der Fumarsäure, der Benzolsulfonsäure, der Camsylsäure oder der Edisylsäure ist; und das Metallsalz ein Calciumsalz, ein Natriumsalz, ein Magnesiumsalz, ein Strontiumsalz oder ein Kaliumsalz ist.

4. Pharmazeutische Zusammensetzung nach Anspruch 1, wobei die Zusammensetzung mit der Formel (I) in einer Menge enthalten ist, die von 0,1 mg bis zu 1.000 mg pro 1 Einheit der Zusammensetzung reicht.

5. Pharmazeutische Zusammensetzung nach Anspruch 1, wobei der saure Zusatzstoff in einer Menge enthalten ist, die von 0,1 bis 100 Gewichtsanteilen reicht, basierend auf 1 Gewichtsanteil der Zusammensetzung mit der Formel (I).

6. Pharmazeutische Zusammensetzung nach Anspruch 5, wobei der saure Zusatzstoff in einer Menge enthalten ist, die von 0,25 bis 50 Gewichtsanteilen reicht, basierend auf 1 Gewichtsanteil der Zusammensetzung mit der Formel (I).

7. Pharmazeutische Zusammensetzung nach Anspruch 1, wobei die Zusammensetzung in Form einer Kapsel oder Tablette formuliert ist.

8. Pharmazeutische Zusammensetzung nach Anspruch 1, ferner einen Hilfsstoff umfassend, ausgewählt aus der Gruppe bestehend aus einem Verdünnungsmittel einem Bindemittel, einem Sprengmittel, einem Schmiermittel und einer Mischung aus diesen.

9. Pharmazeutische Zusammensetzung nach Anspruch 8, wobei das Verdünnungsmittel in einer Menge enthalten ist, die von 20 bis 80 Gew.-% reicht, das Bindemittel in einer Menge enthalten ist, die von 1 bis 10 Gew.-% reicht, das Sprengmittel in einer Menge enthalten ist, die von 1 bis 30 Gew.-% reicht, oder das Schmiermittel in einer Menge enthalten ist, die von 0,5 bis 5 Gew.-% reicht, basierend auf dem Gesamtgewicht des Zusammensetzung.

10. Pharmazeutische Zusammensetzung nach Anspruch 1, die mit einem schnell freisetzenden Beschichtungssubstrat, einem darmlöslichen Beschichtungssubstrat, einem Beschichtungssubstrat für verzögerte Freisetzung oder mit einer Mischung daraus beschichtet ist.

11. Pharmazeutische Zusammensetzung nach Anspruch 10, wobei das schnell freisetzende Beschichtungssubstrat Hydroxypropylcellulose, Hydroxypropylmethylcellulose, Polyvinylalkohol, Polyvinylalkohol-Polyethylenglycol-Pfropfpolymer oder eine Mischung daraus ist; das darmlösliche Beschichtungssubstrat (Metha)acrylatcopolymer, Hydroxypropylmethylcellulosephthalat, Celluloseacetatphthalat oder eine Mischung daraus ist; und das Beschichtungssubstrat für verzögerte Freisetzung Celluloseacetat, Ethylcellulose, Polyvinylacetate oder eine Mischung daraus ist.

12. Pharmazeutische Zusammensetzung nach Anspruch 10, wobei das Beschichtungssubstrat in einer Menge eingesetzt wird, die von 1 bis 50 Gew.-% reicht, basierend auf dem Gesamtgewicht des unbeschichteten Kerns.

## Revendications

1. Composition pharmaceutique comprenant un composé de formule (I) ou son sel acceptable au plan pharmaceutique ; et un additif acide : 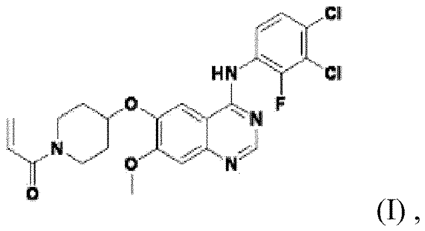 dans laquelle ledit additif acide est l'acide alginique ou SiO₂.

2. Composition pharmaceutique selon la revendication 1, dans laquelle le sel acceptable au plan pharmaceutique est un sel d'acide inorganique, un sel d'acide organique ou un sel métallique.

3. Composition pharmaceutique selon la revendication 2, dans laquelle le sel d'acide inorganique est un sel d'acide chlorhydrique, d'acide phosphorique, d'acide sulfurique ou d'acide disulfurique ; le sel d'acide organique est un sel d'acide malique, d'acide maléique, d'acide citrique, d'acide fumarique, d'acide bésylique, d'acide camsylique ou d'acide édisylique ; et le sel métallique est un sel de calcium, un sel de sodium, un sel de magnésium, un sel de strontium ou un sel de potassium.

4. Composition pharmaceutique selon la revendication 1, dans laquelle le composé de formule (I) est présent en quantité allant de 0,1 mg à 1000 mg pour 1 unité de la composition.

5. Composition pharmaceutique selon la revendication 1, dans laquelle l'additif acide est présent en quantité allant de 0,1 à 100 parties en poids sur la base de 1 partie en poids du composé de formule (I).

6. Composition pharmaceutique selon la revendication 5, dans laquelle l'additif acide est présent en quantité allant de 0,25 à 50 parties en poids sur la base de 1 partie en poids du composé de formule (I).

7. Composition pharmaceutique selon la revendication 1, dans laquelle la composition se présente sous la forme d'une capsule ou d'un comprimé.

8. Composition pharmaceutique selon la revendication 1, qui comprend en outre un excipient choisi dans le groupe constitué d'un diluant, d'un liant, d'un désintégrant, d'un lubrifiant ou d'un de leurs mélanges.

9. Composition pharmaceutique selon la revendication 8, dans laquelle le diluant est présent en quantité allant de 20 à 80 % en poids, le liant est présent en quantité de 1 à 10 % en poids, le désintégrant est présent en quantité de 1 à 30 % en poids ou le lubrifiant est présent en quantité allant de 0,5 à 5 % en poids sur la base du poids total de la composition.

10. Composition pharmaceutique selon la revendication 1, qui est revêtue d'un substrat d'enrobage à libération rapide, d'un substrat d'enrobage entérique, d'un substrat d'enrobage à libération prolongée ou d'un de leurs mélanges.

11. Composition pharmaceutique selon la revendication 10, dans laquelle le substrat d'enrobage à libération rapide est l'hydroxypropylcellulose, l'hydroxypropylméthyl-cellulose, l'alcool polyvinylique, un polymère greffé d'alcool polyvinylique et de polyéthylène-glycol ou un de leurs mélanges ; le substrat d'enrobage entérique est un copolymère de (méth)acrylate, le phtalate d'hydroxypropyl-méthylcellulose, l'acétate phtalate de cellulose ou un de leurs mélanges ; et le substrat d'enrobage à libération prolongée est l'acétate de cellulose, l'éthylcellulose, le poly(acétate de vinyle) ou un de leurs mélanges.

12. Composition pharmaceutique selon la revendication 10, dans laquelle le substrat d'enrobage est utilisé en quantité allant de 1 à 50 % en poids sur la base du poids total du noyau non enrobé.
